(19) Europäisches Patentamt
European Patent Office
Office européen des brevets

(11) Veröffentlichungsnummer: **0 437 690 B1**

(12) # EUROPÄISCHE PATENTSCHRIFT

(45) Veröffentlichungstag der Patentschrift: **23.11.94**

(51) Int. Cl.$^5$: **C07B 53/00**, C07D 307/33, C07C 69/675

(21) Anmeldenummer: **90120856.1**

(22) Anmeldetag: **31.10.90**

(54) **Verfahren zur asymmetrischen Hydrierung von alpha-Ketocarbonyl-verbindungen zu optisch aktiven alpha-Hydroxycarbonylverbindungen.**

(30) Priorität: **16.01.90 DE 4001019**

(43) Veröffentlichungstag der Anmeldung:
**24.07.91 Patentblatt 91/30**

(45) Bekanntmachung des Hinweises auf die Patenterteilung:
**23.11.94 Patentblatt 94/47**

(84) Benannte Vertragsstaaten:
**BE CH DE FR GB IT LI**

(56) Entgegenhaltungen:
**EP-A- 0 218 970**
**EP-A- 0 251 164**
**EP-A- 0 301 457**

(73) Patentinhaber: **Degussa Aktiengesellschaft**
**Weissfrauenstrasse 9**
**D-60311 Frankturt (DE)**

(72) Erfinder: **Schäfer, Adolf, Dr.**
**Nussbaumplatz 6**
**W-6000 Frankturt 60 (DE)**
Erfinder: **Arntz, Dietrich, Dr.**
**Lorsbachstrasse 32**
**W-6370 Oberursel (DE)**

**EP 0 437 690 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur asymmetrischen Hydrierung von α-Ketocarbonylverbindungen aus der Gruppe der α-Ketocarbonsäuren, α-Ketocarbonsäureester, α-Ketocarbonsäureamide und insbesondere α-Ketolactone zu den entsprechenden optisch aktiven α-Hydroxycarbonylverbindungen in Gegenwart von chiralen Iridiumkomplexen, welche einen optisch aktiven 1,2-Diphosphanliganden enthalten, als Katalysator und einem Cokatalysator aus der Gruppe cyclischer Dicarbonsäureimide. Die Erfindung richtet sich ganz besonders auf die Herstellung von (R)-(-)-Pantolacton ((R)-(-)-3,3-Dimethyl-2-hydroxy-γ -butyrolacton) aus Ketopantolacton (Dihydro-4,4-dimethyl-2,3-furandion) durch asymmetrische Hydrierung.

Die Hestellung optisch aktiver α-Hydroxycarbonylverbindungen aus den entsprechenden α-Ketocarbonylverbindungen durch asymmetrische Hydrierung unter Verwendung von chiralen Platinmetall-diphosphankomplexen, insbesondere Rhodium(I)-1,4-diphosphankomplexen, ist eingehend untersucht worden. So lehrt die GB-PS 1 592 536 die asymmetrische Hydrierung von α-Ketocarbonylverbindungen wie z. B. Brenztraubensäurealkylester, Phenyl- und Alkylglyoxylsäurealkylester und Ketopantolacton in Gegenwart von Rhodiumkomplexen mit einem optisch aktiven 1,4-Diphosphanliganden auf der Basis eines 4-Diarylphosphino-2-diarylphosphinomethyl-pyrrolidins oder eines 4,5-Bis(diarylphosphinomethyl)-1,3-dioxolans, das in 2-Stellung auch substituiert sein kann. Weitere Publikationen wie z. B. die EP-A 0 158 875, EP-A 0 218 970, EP-A 0 251 164, DE-OS 33 02 697, US-PS 4,343,741, Chemistry Letters (The Chem. Soc. of Japan), (1978) Seite 297-298, (1984) Seite 1603-1606 sowie (1986) Seite 2061-2064 und Tetrahedron Letters 28 (1987) Seite 3675-3678 richten sich auf die weitere Ausgestaltung des vorgenannten Verfahrens und die Gewinnung der chiralen 1,4-Diphosphane sowie der diese enthaltenden Rhodiumkomplexe. Über den Mechanismus der asymmetrischen Induktion mittels kationischer und in situ gebildeter anionischer Rh-(I)-komplexe berichten I. OJIMA in J. Organomet. Chem. 195 (1985) S. 239-248. Neben dem chiralen Diphosphan enthielten die Komplexe Diolefine und/oder das Lösungsmittel als Stützliganden sowie ein koordinierendes oder nicht-koordinierendes Anion.

Das vorbekannte Verfahren unter Verwendung von Rhodiumkomplexen mit einem chiralen 1,4-Diphosphanliganden erlaubt zwar die Gewinnung von α-Hydroxycarbonylverbindungen, insbesondere (R)-(-)-Pantolacton, in hoher Ausbeute und zum Teil hoher optischer Ausbeute, jedoch sind die besonders gut wirksamen optisch aktiven 1,4-Diphosphane auf Pyrrolidinbasis nur in vielstufigen Synthesen zugänglich - vgl. beispielsweise EP-A 0 251 164 - wodurch die Wirtschaftlichkeit des Verfahrens erheblich beeinträchtigt wird.

Aus der EP-A 0 151 282 und EP-A 0 185 882 sind optisch aktive 3,4-Bis(diphenylphosphino)-pyrrolidine als leicht zugängliche 1,2-Diphosphane für chirale Rhodiumkomplexe für die asymmetrische Hydrierung bekannt. Die genannten Komplexe wurden als Katalysatoren zur asymmetrischen Hydrierung von α-Acylaminoacrylsäuren vorgeschlagen. Bei der Verwendung der genannten und anderer chiraler 1,2-Diphosphane als Ligand in Rhodiumkomplexen mit Dien-Stützliganden zur asymmetrischen Hydrierung von Ketopantolacton konnte jedoch (R)-(-)-Pantolacton nur in niedriger optischer Ausbeute gewonnen werden; zudem waren die Umsätze gering - siehe Vergleichsbeispiel 1.

Die Verwendung von chiralen 1,2-Diphosphanen in Komplexen, welche Iridium als Zentralatom anstelle Rhodium enthalten, führte bei der Hydrierung von Ketopantolacton gleichfalls nur zu unbefriedigenden Umsätzen und optischen Ausbeuten - siehe Vergleichsbeispiel 2.

In die Untersuchungen von I. OJIMA (siehe oben) zum Mechanismus der asymmetrischen Hydrierung von α-Ketocarbonylverbindungen wurde auch ein Iridiumkomplex, nämlich Ir(DIOP)(COD)Cl (COD = 1,5-Cyclooctadien: DIOP = (-)-4,5-Bis(diphenylphosphino)-2,2-dimethyl-1,3-dioxolan) einbezogen - vgl. S. Brunie, J. Mazan, N. Langlois und H.B. Kagan J. Organomet. Chem. 114, 225 (1976). Hiernach wurde von der Konformation des Ir-Komplexes angenommen, daß sie kaum asymmetrisch induzierend sei. Von der Anmelderin wurden mit dem genannten Komplex eine mäßige optische Induktion und geringe Umsätze festgestellt.

Die Aufgabe der Erfindung richtet sich somit auf die Bereitstellung eines Verfahren zur asymmetrischen Hydrierung von α-Ketocarbonylverbindungen aus der Gruppe der α-Ketocarbonsäuren, α-Ketocarbonsäureester, α-Ketocarbonsäureamide und insbesondere Ketolactone zu den entsprechenden optisch aktiven α-Hydroxycarbonylverbindungen in Gegenwart von chiralen Platinmetall-diphosphankomplexen als Katalysator, das es gestattet, optisch aktive 1,2-Diphosphane als chiralen Liganden zu verwenden. Insbesondere bestand ein Interesse, auch mit besonders einfach zugänglichen 1,2-Diphosphanen die α-Hydroxycarbonylverbindungen, ganz besonders (R)-(-)-Pantolacton, in hoher Ausbeute und in hoher optischer Ausbeute zu gewinnen. Die Hydrierung sollte auch bei hohem Druck ohne Verlust an Enantioselektivität und Katalysatoraktivität durchgeführt werden können.

Die Aufgabe wird dadurch gelöst, daß man chirale Iridiumkomplexe verwendet, welche einen optisch aktiven 1,2-Diphosphanliganden der allgemeinen Formel Ia, Ib oder Ic

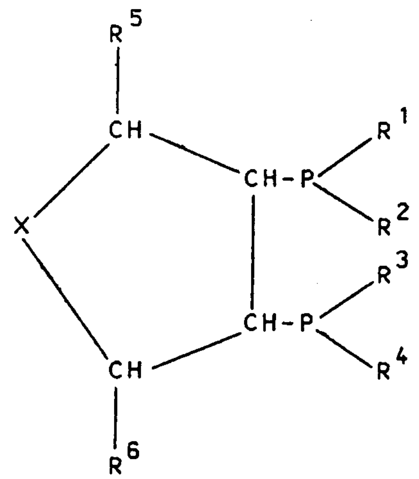

(Ia),

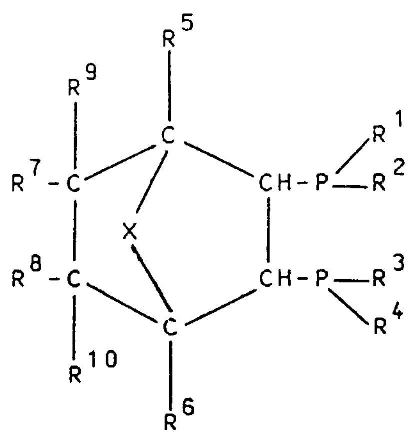

(Ib),

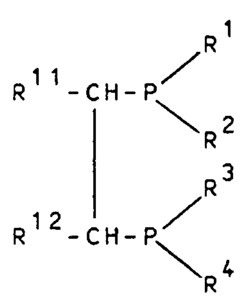

(Ic)

enthalten, worin
$R^1$, $R^2$, $R^3$ und $R^4$ gleich oder verschieden sind und $C_1$- bis $C_8$-Alkyl, das linear oder verzweigt sein kann, Cyclohexylmethyl, $C_5$- bis $C_7$-Cycloalkyl, das ein oder zwei $C_1$- bis $C_4$-Alkylsubstituenten aufweisen kann, Benzyl oder Phenyl bedeuten, wobei der aromatische Ring $C_1$- bis $C_4$-Alkyl-, $C_1$- bis $C_4$-Alkoxy-, Di-($C_1$- bis $C_4$-)alkylamino- oder $C_1$- bis $C_4$-Alkyloxycarbonyl-Substituenten enthalten kann oder $R^1R^2$ oder/und $R^3R^4$ eine ortho-Biphenylengruppe darstellen,
X für Sauerstoff, die Gruppe $NR^{13}$, $CR^{14}R^{15}$, $-CR^{14}=CR^{15}-$ oder $-CHR^{14}-CHR^{15}-$ steht, worin $R^{13}$ Wasser-

stoff, $C_1$-bis $C_6$-Alkyl, $C_5$- bis $C_7$-Cycloalkyl, Benzyl oder eine Acylgruppe aus der Reihe -COAlkyl, -COAryl, -COOAlkyl, -COOAryl, $-SO_2$Aryl, -P(O)$Aryl_2$, bedeutet, wobei die Alkylgruppe 1 bis 4 C-Atome enthält und Aryl für Phenyl oder Naphthyl steht und einen oder zwei $C_1$- bis $C_4$-Alkyl-, $C_1$- bis $C_4$-Alkoxy-, Di-($C_1$-bis $C_4$)alkylamino-, $C_1$- bis $C_4$-Alkoxycarbonylsubstituenten enthalten kann, und $R^{14}$ und $R^{15}$ gleich oder verschieden sind und Wasserstoff, $C_1$- bis $C_4$-Alkyl oder Phenyl bedeuten.

$R^5$ und $R^6$ gleich oder verschieden sind und Wasserstoff, $C_1$- bis $C_4$-Alkyl oder Phenyl bedeuten.

$R^7$ und $R^8$ gleich oder verschieden sind und Wasserstoff, Phenyl, $C_1$- bis $C_4$-Alkoxycarbonyl oder $C_1$-bis $C_4$-Alkyl bedeuten.

$R^{11}$ und $R^{12}$ gleich oder verschieden sind und $C_1$- bis $C_6$-Alkyl, Benzyl oder Phenyl bedeuten, einer der Reste $R^{11}$ und $R^{12}$ auch Wasserstoff sein kann oder, unter der Voraussetzung, daß mindestens ein Phosphoratom oder einer der Reste $R^1$ bis $R^4$ chiral ist, auch $R^{11}$ und $R^{12}$ für Wasserstoff steht, und die Hydrierung in Gegenwart eines Cokatalysators aus der Gruppe cyclischer Dicarbonsäureimide der allgemeinen Formel II

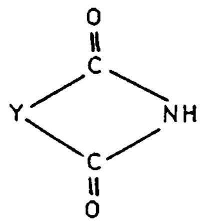

(II),

worin Y eine Ethylen- oder Tri- oder Tetramethylgruppe, welche einen Phenylrest oder einen oder zwei $C_1$-bis $C_8$-Alkylsubstituenten aufweisen können, sowie eine Vinylen- oder ortho-Arylengruppe, welche ein oder zwei Substituenten aus der Gruppe ($C_1$-$C_4$)-Alkyl, ($C_1$-$C_4$)-Alkoxy, ($C_1$-$C_4$)-Alkoxycarbonyl, Di-($C_1$-$C_4$)-alkylamino enthalten kann, bedeuten, durchführt.

Die Unteransprüche richten sich auf bevorzugte Ausführungsformen.

Der Kern der Erfindung ist in der Verwendung der sowohl hinsichtlich der Hydrieraktivität als auch der asymmetrischen Induktion sehr wirksamen Kombination eines chiralen Iridium komplexes mit einem optisch aktiven 1,2-Diphosphanliganden als Katalysator mit einem cyclischen Dicarbonsäureimid der allgemeinen Formel II zu sehen. Wie aus den Vergleichsbeispielen 1 und 2 folgt, bleiben bei der asymmetrischen Hydrierung der untersuchten $\alpha$-Ketocarbonylverbindungen sowohl der Umsatz als auch die optische Ausbeute weit unter den für technische Zwecke angestrebten Werten, wenn die chiralen Ir- bzw. Rh-komplexe in Abwesenheit des Cokatalysators verwendet werden. Die Wirkung der als Cokatalysator eingesetzten cyclischen Dicarbonsäureimide war nicht vorhersehbar.

Nach dem in der EP-A 0 301 457 beschriebenen Verfahren lassen sich Imine mit chiralen Iridium(dien)-(1,2-diphosphan)-komplexen asymmetrisch hydrieren. Gemäß der bevorzugten Ausführungsform werden zusätzlich Ammoniumhalogenide, insbesondere Jodide, welche offensichtlich eine cokatalysierende Wirkung entfalten, eingesetzt. Die Mitverwendung derartiger Stoffe gestatten bei der asymmetrischen Hydrierung von $\alpha$-Ketocarbonylverbindungen praktisch keine Steigerung der optischen Ausbeute.

Die erfindungsgemäßen Cokatalysatoren üben sowohl auf kationische als auch neutrale Iridiumkomplexe der allgemeinen Struktur $[Ir(1,2\text{-}DP)L_2]^+A^-$ bzw. $[Ir(1,2\text{-}DP)(L)_2(Z)]$, wobei 1,2-DP ein optisch aktives 1,2-Diphosphan, L ein Stützligand, Z ein koordinierendes Anion bedeuten, eine kräftige cokatalysierende Wirkung aus. Erst durch die Anwesenheit der Cokatalysatoren gelingt es, hohe optische Ausbeuten zu erzielen, zumindest aber die Enantioselektivität wesentlich zu steigern. Überraschenderweise wird gleichzeitig der Katalysator durch den Cokatalysator aktiviert, wodurch die Hydrierzeiten verkürzt und die Umsätze erhöht werden. Ein weiterer Vorteil der erfindungsgemäßen Katalysator-Cokatalysator-Kombination besteht darin, daß der Katalysator stabilisiert wird, das heißt, auch unter drastischen Hydrierbedingungen, wie insbesondere bei Drucken zwischen 5 und 20 MPa, kommt es nicht zu einer Clusterbildung und damit Inaktivierung des Katalysators oder gar Ausfällung des Iridiums.

Durch Mitverwendung von cyclischen Dicarbonsäureimiden bei der asymmetrischen Hydrierung von $\alpha$-Ketocarbonylverbindungen in Gegenwart der zu den oben genannten Ir-komplexen strukturgleichen Rhodiumkomplexe wird zwar die optische Ausbeute gesteigert - im Falle von Pantolacton von etwa 12 % ee bis auf Werte von 76 % ee (ee bedeutet den Enantiomerenüberschuß) - jedoch schwanken die optischen Ausbeuten ohne bisher erkennbare Gründe stark.

Bei den Katalysatoren handelt es sich um chirale Iridium(I)-komplexe, welche in den üblichen Lösungsmitteln, wie sie für die Hydrierung häufig zum Einsatz kommen, löslich sind. Die Katalysatoren können aber

auch an einem Trägermaterial, etwa einem Kationenaustauscher oder einem Polymeren mit koordinierenden Carboxylatgruppen, gebunden sein. Bevorzugt werden aber lösliche Komplexe, wobei diese in Substanz dem Hydrieransatz zugegeben oder in situ aus einer Katalysatorvorform und einem optisch aktiven Diphosphan gebildet werden können. Außer dem 1,2-Diphosphanliganden (1,2-DP) enthalten die neutralen Ir-Komplexe noch ein Anion Z und zwei Stützliganden L mit je einer Bindungsstelle oder einen Stützliganden mit zwei Bindungsstellen. Bei den Stützliganden handelt es sich um Stoffe mit mindestens einem für Koordinationszwecke geeigneten Elektronenpaar. Die mit dem Katalysator oder der Katalysatorvorform in den Hydrieransatz eingebrachten Stützliganden können ganz oder teilweise durch koordinierende Lösungsmittel, wie z. B. Alkohole, und, so wird angenommen, den Cokatalysator ersetzt werden. Unter den möglichen Stützliganden haben Nitrile, wie Benzonitril oder niedere Alkylnitrile, und Olefine praktische Bedeutung erlangt, wobei niedere Monoolefine und insbesondere Diolefine besonders bevorzugt sind. Bei dem Anion handelt es sich vorzugsweise um Chlorid, Bromid und Jodid. Auch koordinierende Carboxylate, wie sie aus der EP-A 0 218 970 bekannt sind, können verwendet werden. Z gleich Chlorid ist besonders bevorzugt, weil sich die Katalysatoren gut aus Ir(III)-chloridhydrat herstellen lassen.

Die kationischen Ir(I)-Katalysatoren enthalten ebenfalls einen 1,2-Diphosphanliganden (1,2-DP) und zwei Stützliganden L mit der gleichen Bedeutung wie bei den zuvor charakterisierten neutralen Komplexen: bei dem nun nicht koordinierten Anion handelt es sich vorzugsweise um $BF_4^-$, $PF_6^6$, $ClO_4^-$, aber auch $(C_6H_5)_4B^-$, $CF_3SO_3^-$, $SbCl_6^-$, $SbF_6^-$. Besonders bevorzugte Ir(I)-Komplexe sind $[Ir(en)_2(1,2\text{-}DP)(Z)]$ (IIIa) und $[Ir(en)_2(1,2\text{-}DP)]^+A^-$ (IIIb), wobei en, 1,2-DP, Z und A die vorgenannte Bedeutung haben. Besonders bevorzugte Komplexe enthalten als $(en)_2$ ein Dien, insbesondere 1,5-Cycloocadien (COD), 2,5-Norbornadien (NBD) sowie nicht-konjugiertes Hexadien.

Die Ir(I)-Katalysatoren können in an sich bekannter oder in zu strukturgleichen Rhodiumkomplexen analoger Weise hergestellt werden. Es wird hier verwiesen auf z. B. R. Uson et al, Inorg. Chim. Acta 73 (1983), 275 ff; S. Brunie et al, J. Organomet. Chem. 114 (1976), 225-235; M. Green et al, J. Chem. Soc. (A) (1971) 2334 ff; EP-A 0 158 875. Als für die Katalysatorherstellung besonders geeignete Vorstufe haben sich Komplexe vom Typ $[Ir(en)_2Z]_2$, insbesondere etwa $[Ir(COD)Cl]_2$ erwiesen. Durch Zugabe des optisch aktiven Diphosphans zu der genannten Vorstufe erhält man neutrale Komplexe vom Typ der allgemeinen Formel IIIa. Fügt man nach dem Diphosphan noch ein Alkali-oder Ammoniumsalz mit einem nicht-koordinierenden Anion A zu einer Lösung von $[Ir(en)_2Z]_2$, so kann der kationische Katalysator vom Typ der allgemeinen Formel IIIb gewonnen werden.

Bei dem Cokatalysator der allgemeinen Formel II handelt es sich um cyclische Dicarbonsäureimide mit 5 bis 7 Ringgliedern. Der Ring kann gesättigt sein, wie im Falle des ganz besonders bevorzugten Succinimids. Der Ring kann aber auch eine Doppelbindung enthalten, wie im Falle des Maleinimids oder Phthalimids. Die ringbildenden Glieder der Gruppe Y mit 2 bis 4 C-Atomen können alkylsubstituiert sein. Unter den Cokatalysatoren aus der Reihe der Aryl-orthodicarbonsäureimide, worin Y in der Formel II eine ggf. ein- oder zweifach substituierte ortho-Arylengruppe bedeutet, werden ortho-Phthalimid, 1,2-, 2,3- oder 1,8-Naphthalindicarbonsäureimid bevorzugt.

Der Ir-Katalysator und der Cokatalysator werden im Molverhältnis 1:1 bis 1:50, vorzugsweise 1:2 bis 1:25 und insbesondere 1:3 bis 1:6, bei der Hydrierung eingesetzt. Das Molverhältnis des Ir-Katalysators zu dem zu hydrierenden Substrat wird im allgemeinen im Bereich von 1:100 bis 1:10.000, vorzugsweise 1:200 bis 1:2.000, liegen.

Die meisten der im erfindungsgemäßen Verfahren zu verwendenden optisch aktiven 1,2-Diphosphane der allgemeinen Formeln Ia, Ib und Ic sind bekannt. Einzelne Vertreter dieser Stoffklassen, soweit sie nicht per se bereits bekannt sind, lassen sich in an sich bekannter Weise herstellen. Es wird hier verwiesen auf H.B. Kagan "Chiral Ligands for Asymmetric Catalysis" in Asymmetric Synthesis, Vol. 5 (1985), Seite 13-23; EP-A 0 151 282 und EP-A 0 185 882. Die 1,2-Diphosphane können ein oder mehrere Chiralitätszentren aufweisen. Prinzipiell können ein oder beide Phosphoratome chiral sein, bevorzugt befinden sich ein oder zwei Chiralitätszentren in dem Molekülteil, das die $PR^1R^2$- und $PR^3R^4$-Gruppen in vicinaler Stellung aufweisen. Bevorzugt werden solche 1,2-Diphosphane, in welchen die Gruppen $PR^1R^2$ und $PR^3R^4$ gleich sind, und somit $R^1=R^3$ und $R^2=R^4$ ist. Besonders gut zugänglich und zugleich sehr wirksam sind 1,2-Diphosphane mit wenigstens einer, vorzugsweise zwei gleichen Arylgruppen pro Phosphoratom, insbesondere Phenyl, sind besonders bevorzugt.

Diphosphorane der allgemeinen Formel Ia mit $R^5=R^6$ und insbesondere $R^5=R^6=H$ sind bevorzugt; dies gilt besonders für die Verbindungen mit $X=NR^{13}$ und zwei gleichen Phosphinogruppen am Pyrolidinring. Optisch aktive 3,4-Bis(diarylphosphino)-pyrrolidine lassen sich in einfacher Weise in wenigen Schritten aus natürlicher Weinsäure gewinnen.

Bei den Diphosphanen der allgemeinen Formel Ib handelt es sich um bicyclische Verbindungen, wie sie beispielsweise durch Diels-Alder-Addition eines Dienophils an ein cyclisches Dien mit vorheriger oder

nachträglicher Einführung der Phosphinogruppen zugänglich sind - vgl. H.B. Kagan, loc. cit. Bevorzugt sind Verbindungen, worin $R^5 = R^6 = R^7 = R^8$ = Wasserstoff und $PR^1R^2 = PR^3R^4$ bedeuten. Besonders vorteilhaft ist die Verwendung von 2,3-Bis(diphenylphosphino)-bicyclo-[2,2,1]-hept-5-en.

Diphosphane vom Typ Ic, in welchen $PR^1R^2$ und $PR^3R^4$ gleich sind, $R^{11}$ Wasserstoff oder Methyl und $R^{12}$ eine lineare oder verzweigte $C_1$- bis $C_4$-Alkylgruppe oder eine Phenyl-, Benzyl oder Cyclohexylgruppe bedeuten, sind bevorzugt. Verbindungen mit $R^{11}$ gleich Wasserstoff lassen sich aus natürlichen α-Aminosäuren oder α-Hydroxycarbonsäuren gewinnen, wobei die α-Aminosäurenzunächst in die α-Hydroxycarbonsäuren überführt und dann in bekannter Weise in die optisch aktiven Diphosphane überführt werden - vgl. H.B. Kagan loc. cit.

Die asymmetrisch zu hydrierenden Substrate, die α-Ketocarbonylverbindungen, können Ester, Amide, Carbonsäuren und insbesondere α-Ketolactone sein. Außer der α-Ketocarbonylgruppierung können sie solche funktionellen Gruppen oder Substituenten enthalten, welche sich unter den gewählten Hydrierbedingungen nicht verändern. Die bevorzugten α-Ketolactone stellen 5- bis 7-gliedrige Ringe dar. Diese Ringe können beispielsweise ein oder mehrere niedere Alkylgruppen mit 1 bis 6 C-Atomen oder Arylgruppen, welche ggf. substituiert sein können, aufweisen. Ein besonders bevorzugtes Substrat ist α-Ketopantolacton.

Der Lactonring kann über ein oder zwei C-Atome auch mit einem weiteren 5- bis 7-gliedrigen Cycloaliphaten verbunden sein. Unter den offenkettigen α-Ketocarbonylverbindungen sind jene mit einer linearen oder verzweigten $C_1$- bis $C_6$-Alkyl-, $C_5$- oder $C_6$-Cycloalkyl- oder Phenylgruppe, welche auch ($C_1$-$C_4$)-Alkyl-, ($C_1$-$C_4$)-Alkoxyl, ($C_1$-$C_4$)-Alkoxycarbonyl, Di-($C_1$-$C_4$)-alkylamino enthalten kann, neben der zu hydrierenden Ketogruppe bevorzugt. Bei den bevorzugten Substituenten an der Ketogruppe benachbarten Carbonylgruppe offenkettiger α-Ketocarbonylverbindungen handelt es sich um Alkoxygruppen mit 1 bis 12 C-Atomen, welche linear, verzweigt oder cyclisch und auch arylsubstituiert sein können; niedere Alkoxygruppen mit 1 bis 4 C-Atomen sind bevorzugt.

Die asymmetrische Hydrierung wird üblicherweise in Gegenwart eines organischen Lösungsmittels für die α-Ketocarbonylverbindung und, sofern keine trägergebundenen Ir-Katalysatoren verwendet werden, für den Katalysator und Cokatalysator durchgeführt. Geeignet sind insbesondere Lösungsmittel aus der Reihe der aromatischen Kohlenwasserstoffe, Ether, $C_1$- bis $C_6$-Alkohole, Halogenkohlenwasserstoffe, welche allein oder in Form von Lösungsmittelgemischen zur Anwendung gelangen. Die bei der asymmetrischen Hydrierung erreichbare optische Ausbeute und auch der Umsatz hängen stark vom Lösungsmittelsystem ab, so daß im Einzelfall ein orientierender Vorversuch angezeigt ist. Gemische aus einem aromatischen Kohlenwasserstoff und einem niederen Alkohol sind besonders vorteilhaft, insbesondere beispielsweise Gemische aus Toluol oder Xylol mit einem $C_3$- bis $C_5$-Alkohol. Wie aus den Beispielen folgt, ist ein Toluol/tert.-Butanol-Gemisch im Volumverhältnis von etwa 1:3 besonders vorteilhaft, wenn α-Ketopantolacton zu (R)-(-)-Pantolacton hydriert werden soll.

Die Hydrierung erfolgt bei Drucken im Bereich von 0,1 bis 20 MPa, vorzugsweise bei 0,5 bis 12 MPa. Durch Druckerhöhung wird die Raum-Zeit-Ausbeute erhöht.

Die Hydriertemperatur erfolgt im allgemeinen bei 10 bis 100 °C, jedoch sind prinzipiell auch tiefere oder höhere Temperaturen möglich. Besonders bevorzugt wird bei 30 bis 70 °C hydriert.

Die Aufarbeitung des hydrierten Reaktionsgemisches erfolgt in dem Fachmann bekannter Weise. Die optische Ausbeute (% ee) kann vorteilhaft gaschromatographisch an Säulen mit chiraler Phase, wie z. B. Chirasil oder Cyclodextrinen, nach Derivatisierung der α-Hydroxycarbonylverbindung erfolgen.

Durch das erfindungsgemäße Verfahren können nun optisch aktive 1,2-Diphosphane, welche bisher nur zu unbefriedigenden Umsätzen und sehr niedrigen optischen Ausbeuten führten, als chirale Liganden in Platinmetall-, insbesondere Iridiumkomplexen, eingesetzt werden. Hierdurch wurden die Möglichkeiten, in Gegenwart chiraler Katalysatoren α-Ketocarbonylverbindungen asymmetrisch zu hydrieren, erweitert.

Die folgenden Beispiele dienen der weiteren Erläuterung der Erfindung.

Nachfolgend gebrauchte Abkürzungen:

| | |
|---|---|
| NBDPP = | (3R,4R)-1-Benzyl-3,4-bis-(diphenylphosphino) pyrrolidin; |
| BDPPP = | (3R,4R)-Bis(diphenylphosphino)pyrrolidin; |
| COD = | 1,5-Cyclooctadien; |
| NBD = | Norbornadien; |
| Norphos = | (2R,3R)-(-)-2,3-Bis(diphenylphosphino) bicyclo- 2,2,1 -hept-5-en |
| (S)/(K) = | Molverhältnis Substrat zu Katalysator |

**Beispiel 1** (Vergleichsbeispiel)

Asymmetrische Hydrierung von Ketopantolacton (Dihydro-4,4-dimethyl-2,3-furandion) mit chiralen Rhodium-1,2-diphosphan-komplexen zu optisch aktivem Pantolacton.

Die Hydrierung erfolgte unter den in der Tabelle angegebenen Bedingungen. Der Katalysator wurde in situ aus $[Rh(en)_2Cl]_2$ und dem Diphosphan gebildet.
siehe Tabelle 1
Aus den Versuchen folgt, daß der Umsatz und die optische Ausbeute sehr niedrig sind, sofern die Hydrierung mit Rh-komplexen in Abwesenheit des erfindungsgemäßen Cokatalysators erfolgt.

**Beispiel 2** (Vergleichsbeispiel)

Asymmetrische Hydrierung von Ketopantolacton mit chiralen Iridium-1,2-diphosphan-komplexen. Zum Einsatz gelangten kationierte Ir-Komplexe, welche in Substanz eingesetzt wurden. Die Versuchsbedingungen und Ergebnisse sind der folgenden Tabelle, die Abkürzungen Beispiel 1 zu entnehmen.
siehe Tabelle 2

Tabelle 1

| Katalysator/ Lösungsmittel | (S)/(K) | Druck MPa | Dauer (h) | Umsatz (%) | Opt. Ausbeute (% ee) | (Konfiguration) |
|---|---|---|---|---|---|---|
| $[Rh(C_2H_4)_2Cl]_2$-NBDPP/Toluol | 205 | 5,3 | 24 | 4,1 | 12,4 | (R) |
| $[Rh(COD)Cl]_2$-NBDPP/Toluol | 217 | 5,0 | 24 | 18,3 | 17,8 | (R) |
| $[Rh(C_2H_4)_2Cl]_2$-BDPPP/Toluol | 202 | 5,0 | 24 | 5,6 | 3,0 | (R) |
| $[Rh(NBD)Cl]_2$-BDPPP/Toluol | 197 | 5,2 | 24 | 8,6 | 2,0 | (R) |
| $[Rh(COD)Cl]_2$-BDPPP/Toluol | 194 | 5,1 | 24 | 6,8 | 8,4 | (R) |
| $[Rh(COD)Cl]_2$-Norphos/Toluol | 202 | 5,1 | 24 | 6,8 | 10,8 | (S) |

Tabelle 2

| Katalysator/ Lösungsmittel | (S)/(K) | Druck (MPa) | Temp. (°C) | Dauer (h) | Umsatz (%) | Opt. Ausbeute (% ee. (R)) |
|---|---|---|---|---|---|---|
| $[Ir(COD)(NBDPP)]$-$BF_4$/Toluol | 205 | 1,0 | 50 | 8-9 | 14,8 | 3,6 - 8,0 |
| $[Ir(COD)(BDPPP)]$-$BF_4$/Toluol | 200 | 1,0 | 50 | 10 | 12,3 | 3,0 |

**Beispiel 3**

a) Allgemeine Arbeitsvorschrift zur Hydrierung mit isolierten, kationischen und neutralen Komplexen

In einem Schlenkrohr werden unter Argon zunächst der Katalysator und der Cokatalysator in 100 ml Lösungsmittel gelöst. Die klare Katalysatorlösung wird dann zu einer ebenfalls unter Argon angesetzten Lösung der α-Ketocarbonylverbindung (40 mMol) in 100 ml Lösungsmittel gegeben. Dieses Gemisch wird dann in einen evakuierten 500 ml-Autoklaven eingesaugt. Nun wird Wasserstoff bis auf den gewünschten Druck aufgedrückt und auf die gewünschte Temperatur hochgeheizt. Über eine Druckhaltevorrichtung wird ein konstanter Druck gehalten und die Druckabnahme am Vorratsgefäß registriert. Nach dem Ende der $H_2$-Aufnahme wird aus dem Reaktionsgemisch in üblicher Weise das Lösungsmittel unter vermindertem Druck abdestilliert. Der Rückstand wird dann einer Feststoff-Destillation im Ölpumpenvakuum unterworfen, um das hydrierte Produkt vom Katalysator abzutrennen. Das erhaltene Produkt wird dann über $^1H$-NMR, Gaschromatographie und spezifische Drehung charakterisiert.

b) Allgemeine Arbeitsvorschrift zur Hydrierung mit in situ Katalysatoren

In einem 500 ml 2 Halskolben werden 40 mMol der $\alpha$-Ketocarbonylverbindung vorgelegt, evakuiert und mit Argon beaufschlagt. Dann werden 150 ml des Lösungsmittels oder des Lösungsmittelgemisches und anschließend der Cokatalysator hinzugefügt. In einem mit Argon inertisierten Schlenkrohr werden im Falle eines Substrat-/Komplex-Molverhältnisses von 200:1 0,1 mMol Ir(1,5-COD)Cl $_2$ und 0,204 mMol Ligand vorgelegt und mit 50 ml Lösungsmittel oder Lösungsmittelgemisch versetzt. Man rührt die Mischung ca. 30 Minuten, bis sich eine homogene gelbe bis orange Lösung gebildet hat. Nach Vereinigen der Komplexlösung mit der Substratlösung in einem 500 ml-Autoklaven wird wie unter a) beschrieben hydriert und danach aufgearbeitet.

c) Herstellung von kationischen Ir-Komplexen:

[Ir(1,5 COD)((R,R)-NBDPP)]$BF_4$:

1,3 g [Ir(1,5-COD)Cl]$_2$ (1,94 mMol) und 2,07 g (RR)-1-Benzyl-3,4-bis(diphenylphosphino)pyrrolidin (=NBDPP) (3,9 mMol) werden unter Argon bei Raumtemperatur in 50 ml Methanol abs. gelöst. Die sich bildende tiefrote Lösung wird 0,5 h gerührt. Anschließend tropft man eine Lösung von 0,9 g $NaBF_4$ (8,2 mMol) in 50 ml Wasser ($O_2$-frei) über 1,5 h zu. Es fällt ein rotvioletter Niederschlag aus. Diese Suspension wird noch 1 h weitergerührt und anschließend über eine Fritte filtriert. Man wäscht mit 1 x 20 ml Wasser ($O_2$-frei) und 3 x mit je 5 ml Ether abs. Nach Trocknung im Vakuum wird aus 10 ml Methylenchlorid abs. und 20 ml Ether abs. umkristallisiert. Ausbeute: 3,3 g rotviolette Kristalle (92,4 % der Theorie); Schmelzpunkt 185 °C (Zers.). Der Komplex wurde mittels IR-, $^1H$- und $^{31}P$-NMR- sowie Massenspektrum charakterisiert.

Kationische Komplexe mit anderen 1,2 Diphosphanen und anderen Stützliganden lassen sich in vergleichbarer Weise herstellen.

[Ir (1,5 COD)(RR)-NBDPP)]$PF_6$: Dieser Komplex läßt sich in gleicher Weise herstellen, indem $NH_4PF_6$ anstelle $NaBF_4$ verwendet wird. Schmelzpunkt 215 °C (Zers.).

[Ir(1,5 COD)((R,R)-BDPPP)]$BF_4$:

1,03 g [Ir(1,5-COD)Cl]$_2$ (1,53 mMol) und 1,34 g (RR)-3,4-Bis(diphenylphosphino)-pyrrolidin (=BDPPP) (3,9 mMol) werden unter Argon bei 0 °C in 70 ml Methanol abs. gelöst. Die sich bildende rotviolette Lösung wird sofort mit 0,6 g $NaBF_4$ (5,46 mMol), gelöst in 90 ml Wasser ($O_2$-frei), versetzt. Es bildet sich ein flockiger grauroter Niederschlag, der nach einer weiteren Stunde Rühren abfiltriert und verworfen wird. Zur Fällung des Produkts wird nun bei 0 °C die rotviolette Mutterlauge mit 100 ml Wasser ($O_2$-frei) versetzt. Dann wird über eine Fritte abgetrennt. Der Rückstand wird mit 15 ml Wasser ($O_2$-frei) gewaschen. Nach Trocknen wird aus Methylenchlorid/Ether umkristallisiert, wobei ein dunkelrotes Pulver erhalten wird, das bei 218 °C unter Zersetzung schmilzt und aufgrund spektroskopischer Untersuchungen als der genannte Komplex charakterisiert wurde.

d) Herstellung von neutralen Ir-Komplexen:

[Ir(1,5-COD)((R,R)-NBDPP)Cl]:

2,4 g[Ir(1,5-COD)Cl]$_2$ (3,57 mMol) werden mit 3,97 g (R,R)-NBDPP (7,5 mMol) in einem Schlenkrohr vorgelegt, evakuiert und unter Argon mit 50 ml Ethanol abs. versetzt. Die blasrote Lösung wird über 2 h bei Raumtemperatur gerührt, wobei sich beiger Niederschlag bildet, der über eine Fritte abgetrennt wird. Es wird 3 x mit je 10 ml Ether abs. gewaschen und im Hochvakuum getrocknet. Bei Bedarf wird aus Methylenchlorid/Ether umkristallisiert. Schmelzpunkt 195 °C; Ausbeute 87,3 %. Der Komplex wurde durch IR-, NMR- und Massenspektren charakterisiert.

**Beispiel 4**

Asymmetrische Hydrierungen von Ketopantolacton mit kationischen Ir-Komplexen und Succinimid als Cokatalysatoren. Die Hydrierbedingungen und Ergebnisse sind der Tabelle 3 zu entnehmen; die Hydriertemperatur betrug 50 °C.

siehe Tabelle 3

**Beispiel 5**

Asymmetrische Hydrierung von Ketopantolacton mit neutralen Ir-Komplexen und Succinimid als Cokatalysator. Die Hydrierbedingungen und Ergebnisse sind der Tabelle 4 zu entnehmen; Hydriertemperatur 50 °C.
siehe Tabelle 4

**Beispiel 6**

Asymmetrische Hydrierung von Ketopantolacton mit $[Ir(COD)Cl]_2$ / Diphosphan vom Typ der allgemeinen Formel Ic und Succinimid als Cokatalysator. Hydrierbedingungen: Druck = 1,0 MPa; Temperatur = 50 °C; Dauer = 6 Stunden; (S)/(K) = 200; Lösungsmittel = Toluol/tert. Butanol (1:3); (CK)/(K) = 1:10; eingesetztes Diphosphan und Ergebnisse
siehe Tabelle 5.

Tabelle 3

| Beispiel | Katalysator | Lösungsmittel | (S)/(K) | (CK)/(K) | Druck (MPa) | Dauer (h) | Umsatz (%) | opt. Ausbeute (% ee (R)) |
|---|---|---|---|---|---|---|---|---|
| 4.1 | [Ir(COD)((RR)-NBDPP]$BF_4$ | Toluol | 203 | 10 | 1,0 | 9 | 38,2 | 84,4 |
| 4.2 | wie 4.1 | Toluol/tert. Butanol (1:3) | 207 | 10 | 1,0 | 1,5 | 100 | 88,8 |
| 4.3 | wie 4.1 | Toluol/tert. Butanol (1:3) | 1102 | 10 | 10,8 | 24 | 100 | 84,6 |
| 4.4 | [Ir(COD)((RR)-BDPPP)]$BF_4$ | Toluol | 226 | 10 | 0,5 | 23 | 28,4 | 83,6 |
| 4.5 | wie 4.4 | Toluol/ Methanol (1:3) | 228 | 10 | 0,5 | 9 | 99,2 | 65,0 |
| 4.6 | wie 4.1 | Methanol | 200 | 5 | 0,5 | 9 | 45,8 | 54,8 |
| 4.7 | wie 4.1 | Toluol/ Methanol (1:1) | 200 | 5 | 0,5 | 9 | 100 | 70,9 |

(S)/(K) = Molverhältnis Substrat : Katalysator

(CK)/(K) = Molverhältnis Cokatalysator : Katalysator

Abkürzungen der Katalysatoren siehe Beispiel 3c

Tabelle 4

| Beispiel | Katalysator | Lösungsmittel | (S)/(K) | (CK)/(K) | Druck (MPa) | Dauer (h) | Umsatz (%) | opt. Ausbeute (% ee (R)) |
|---|---|---|---|---|---|---|---|---|
| 5.1 | $[Ir(COD)Cl]_2$/ 2(R,R)-NBDPP (in situ) | Toluol/tert. Butanol (1:3) | 209 | 10 | 1,0 | 2,5 | 100 | 88,8 |
| 5.2 | wie 5.1 | wie 5.1 | 1028 | 10 | 11,0 | 2,0 | 100 | 85,6 |
| 5.3 | [Ir(COD)((R,R)-NBDPP)Cl] (isoliert) | wie 5.1 | 210 | 10 | 0,5 | 2,5 | 100 | 89,5 |
| 5.4 | wie 5.3 | wie 5.1 | 1004 | 10 | 10,6 | 4,0 | 100 | 82,0 |
| 5.5 | $[Ir(COD)Cl]_2$/ 2(R,R)-BDPPP (in situ) | wie 5.1 | 215 | 10 | 1,0 | 2,5 | 100 | 79,0 |
| 5.6 | wie 5.5 | wie 5.1 | 1014 | 10 | 10,9 | 5,0 | 100 | 84,0 |
| 5.7 | $[Ir(COD)Cl]_2$/ 2(R,R)-Norphos (in situ) | wie 5.1 | 205 | 10 | 1,0 | 7,0 | 100 | 89,2 |
| 5.8 | wie 5.7 | wie 5.1 | 1033 | 10 | 11,1 | 3,0 | 100 | 88,8 |
| 5.9 | wie 5.1 | Toluol/$CH_3OH$ (1:3) | 500 | 10 | 0,5 | 9,0 | 58,7 | 7,8 |
| 5.10 | wie 5.1 | Toluol/n-Butanol (1:3) | 500 | 10 | 0,5 | 9,0 | 10,2 | 57,2 |
| 5.11 | wie 5.1 | Toluol/i-Butanol (1:3) | 500 | 10 | 0,5 | 9,0 | 46,2 | 70,0 |
| 5.12 | wie 5.1 | Toluol/tert. Butanol (1:3) | 500 | 10 | 0,5 | 9,0 | 100 | 86,4 |

Tabelle 5

| Beispiel | opt. aktives Diphosphan (Typ Ic) | Umsatz (%) | opt. Ausbeute (% ee) | (Konfiguration) |
|---|---|---|---|---|
| 6.1 | (2S,3S)-2,3-Bis(diphenylphosphino)-butan | 29,7 | 37,4 | (S) |
| 6.2 | (R)-1,2-Bis(diphenylphosphino)-3-methyl-butan | 45,0 | 23,6 | (R) |
| 6.3 | (R)-1,2-Bis(diphenylphosphino)-propan | 41,7 | 30,6 | (R) |

**Beispiel 7**

Unter Verwendung des Katalysators $[Ir(COD)Cl]_2$ / 2 NBDPP und eines Cokatalysators wurde Ketopantolacton zu Pantolacton hydriert: Hydriertemperatur = 50 °C; Druck = 5,3 MPa; Dauer = 6 Stunden; Lösungsmittel = Toluol/tert. Butanol (1:3); Substrat : Katalysator (Molverhältnis) 500:1; Cokatalysator : Katalysator (Molverhältnis) 10:1. Cokatalysator und Ergebnisse siehe Tabelle 6.

Tabelle 6

| Cokatalysator | Umsatz | opt. Ausbeute (% ee) | (Konformation) |
|---|---|---|---|
| Succinimid | 100 | 80,4 | (R) |
| Phthalimid | 97 | 58,8 | (R) |
| Glutarimid | 64,3 | 8,2 | (S) |

**Beispiel 8**

Analog Beispiel 5 wurde Brenztraubensäureethylester unter Verwendung des in situ hergestellten Katalysators $[Ir(COD)Cl]_2$ / 2 NBDPP und Succinimid als Cokatalysator 23 Stdn. bei 50 °C hydriert. Die Betriebsbedingungen und Ergebnisse folgen aus Tabelle 7.

Tabelle 7

| Beispiel | Lösungsmittel | (S)/(K) | (CK)/(K) | Druck (MPa) | Umsatz (%) | opt. Ausbeute (% ee (R)) |
|---|---|---|---|---|---|---|
| 8.1 | Toluol | 203 | ohne CK | 5,4 | 50 | 5,6 |
| 8.2 | Toluol | 203 | 1,0 | 5,5 | 55 | 10,8 |
| 8.3 | Toluol/tert. | 198 | 10,0 | 10,8 | 99 | 10,4 |

**Patentansprüche**

1. Verfahren zur asymmetrischen Hydrierung von α-Ketocarbonylverbindungen aus der Gruppe der α-Ketocarbonsäuren, α-Ketocarbonsäureester, α-Ketocarbonsäureamide und insbesondere α-Ketolactone zu den entsprechenden optisch aktiven α-Hydroxycarbonylverbindungen in Gegenwart von chiralen Platinmetall-diphosphankomplexen als Katalysator,

dadurch gekennzeichnet,
daß man chirale Iridiumkomplexe verwendet, welche einen optisch aktiven 1,2-Diphosphanliganden der allgemeinen Formel Ia, Ib oder Ic

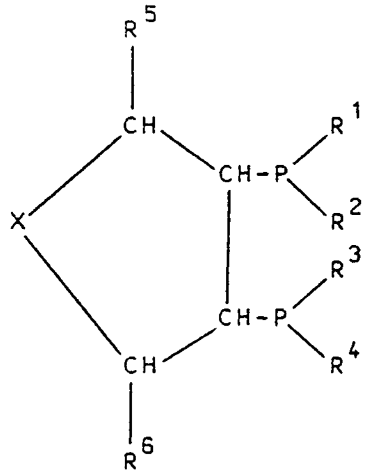

(Ia),

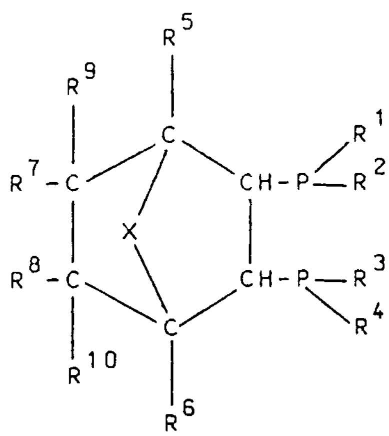

(Ib)

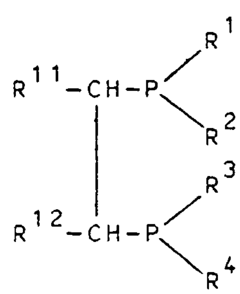

(Ic)

enthalten, worin
$R^1$, $R^2$, $R^3$ und $R^4$ gleich oder verschieden sind und $C_1$- bis $C_8$-Alkyl, das linear oder verzweigt sein kann, Cyclohexylmethyl, $C_5$- bis $C_7$-Cycloalkyl, das ein oder zwei $C_1$- bis $C_4$-Alkylsubstituenten aufweisen kann, Benzyl oder Phenyl bedeuten, wobei der aromatische Ring $C_1$- bis $C_4$-Alkyl-, $C_1$-bis $C_4$-Alkoxy-, Di-($C_1$- bis $C_4$-)alkylamino- oder $C_1$- bis $C_4$-Alkyloxycarbonyl-Substituenten enthalten kann oder $R^1R^2$ oder/und $R^3R^4$ eine ortho-Biphenylengruppe darstellen,
X für Sauerstoff, die Gruppe $NR^{13}$, $CR^{14}R^{15}$, $-CR^{14}=CR^{15}-$ oder $-CHR^{14}-CHR^{15}$ steht, worin $R^{13}$

Wasserstoff, $C_1$-bis $C_6$-Alkyl, $C_5$- bis $C_7$-Cycloalkyl, Benzyl, oder eine Acylgruppe aus der Reihe -COAlkyl, -COAryl, -COOAlkyl, -COOAryl, $-SO_2Aryl$, $-P(O)Aryl_2$, bedeutet, wobei die Alkylgruppe 1 bis 4 C-Atome enthält, und Aryl für Phenyl oder Naphthyl steht, und einen oder zwei $C_1$- bis $C_4$-Alkyl-, $C_1$- bis $C_4$-Alkoxy-, Di-($C_1$- bis $C_4$)alkylamino-, $C_1$- bis $C_4$-Alkoxycarbonylsubstituenten enthalten kann, und $R^{14}$ und $R^{15}$ gleich oder verschieden sind und Wasserstoff, $C_1$- bis $C_4$-Alkyl oder Phenyl bedeuten,
$R^5$ und $R^6$ gleich oder verschieden sind und Wasserstoff, $C_1$- bis $C_4$-Alkyl oder Phenyl bedeuten,
$R^7$ und $R^8$ gleich oder verschieden sind und Wasserstoff, Phenyl, $C_1$- bis $C_4$-Alkoxycarbonyl oder $C_1$- bis $C_4$-Alkyl bedeuten,
$R^9$ und $R^{10}$ gemeinsam eine Doppelbindung darstellen oder Wasserstoff bedeuten,
$R^{11}$ und $R^{12}$ gleich oder verschieden sind und $C_1$- bis $C_6$-Alkyl, Benzyl oder Phenyl bedeuten, einer der Reste $R^{11}$ und $R^{12}$ auch Wasserstoff sein kann oder, unter der Voraussetzung, daß mindestens ein Phosphoratom oder einer der Reste $R^1$ bis $R^4$ chiral ist, auch $R^{11}$ und $R^{12}$ für Wasserstoff steht, und die Hydrierung in Gegenwart eines Cokatalysators aus der Gruppe cyclischer Dicarbonsäureimide der allgemeinen Formel II

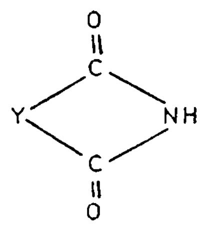

(II),

worin Y eine Ethylen- oder Tri- oder Tetramethylengruppe, welche einen Phenylrest oder einen oder zwei $C_1$- bis $C_8$-Alkylsubstituenten aufweisen können, sowie eine Vinylen- oder ortho-Arylengruppe, welche ein oder zwei Substituenten aus der Gruppe ($C_1$-$C_4$)-Alkyl, ($C_1$-$C_4$)-Alkoxy, ($C_1$-$C_4$)-Alkoxycarbonyl, Di-($C_1$-$C_4$)-alkylamino enthalten kann, bedeuten, durchführt.

**2.** Verfahren nach Anspruch 1,
dadurch gekennzeichnet,
daß man Iridiumkomplexe der allgemeinen Formel IIIa oder IIIb

$[Ir(en)_2(1,2\text{-DP})]^+ A^-$ (IIIa)

$[Ir(Z)(en)_2(1,2\ DP)]$ (IIIb)

als Katalysator verwendet, worin 1,2-DP einen optisch aktiven 1,2-Diphosphanliganden der allgemeinen Formel Ia, Ib oder Ic, $(en)_2$ zwei Moleküle eines Monoolefins oder ein Molekül eines Diolefins, $A^-$ ein nichtkoordinierendes Anion, insbesondere $BF_4^-$, $PF_6^-$, $ClO_4^-$ und Z ein koordinierendes Anion, insbesondere Chlorid oder Carboxylat, das auch in Form eines Ionenaustauschers vorliegen kann, bedeuten.

**3.** Verfahren nach Anspruch 1 oder 2,
dadurch gekennzeichnet,
daß man 1,2-Diphosphanliganden der allgemeinen Formel Ia, Ib oder Ic, in welchen $R^1 = R^3$ und $R^2 = R^4$ und vorzugsweise $R^1 = R^2 = R^3 = R^4$ ist und Phenylreste besonders bevorzugt sind, verwendet.

**4.** Verfahren nach einem oder mehreren der Ansprüche 1 bis 3,
dadurch gekennzeichnet,
daß man 1,2-Diphosphanliganden der allgemeinen Formel Ia verwendet, worin $R^5$ und $R^6$ Wasserstoff und X die Gruppe $NR^{13}$ bedeuten, wobei $R^{13}$ die vorgenannte Bedeutung hat und $R^1 = R^3$ und $R^2 = R^4$ oder vorzugsweise $R^1$, $R^2$, $R^3$ und $R^4$ gleich sind.

**5.** Verfahren nach einem oder mehreren der Ansprüche 1 bis 3,
dadurch gekennzeichnet,
daß man 1,2-Diphosphanliganden der allgemeinen Formel Ib verwendet, worin $R^5$, $R^6$, $R^7$ und $R^8$ Wasserstoff und X eine Methylengruppe bedeuten und $R^1 = R^3$ und $R^2 = R^4$ oder vorzugsweise $R^1$, $R^2$,

$R^3$ und $R^4$ gleich sind.

6. Verfahren nach einem oder mehreren der Ansprüche 1 bis 3, dadurch gekennzeichnet, daß man 1,2-Diphosphanliganden der allgemeinen Formel Ic verwendet, worin $R^{11}$ Wasserstoff oder eine Methylgruppe und $R^{12}$ eine $C_1$- bis $C_4$-Alkylgruppe, welche linear oder verzweigt sein kann, bedeuten.

7. Verfahren nach einem oder mehreren der Ansprüche 1 bis 6, dadurch gekennzeichnet, daß man als Cokatalysator Succinimid in einer Menge von 1 bis 50 Mol, vorzugsweise 2 bis 25 Mol, pro Mol Iridiumkomplex einsetzt.

8. Verfahren nach einem oder mehreren der Ansprüche 1 bis 7, dadurch gekennzeichnet, daß man Ketopantolacton zu (R)-(-)-Pantolacton hydriert.

9. Verfahren nach einem oder mehreren der Ansprüche 1 bis 8, dadurch gekennzeichnet, daß man zunächst den wirksamen Katalysator der allgemeinen Formel IIIb in situ aus einem Ir-Komplex der Formel $[Ir\ (en)_2\ (Z)]_2$, worin $(en)_2$ und Z die vorgenannte Bedeutung haben, durch Zugabe des optisch aktiven 1,2-Diphosphans der Formel Ia, Ib oder Ic in wenigstens einem für die Hydrierung verwendeten Lösungsmittel herstellt und die Ketocarbonylverbindung in Gegenwart der so gewonnenen Katalysatorlösung hydriert.

10. Verfahren nach einem oder mehreren der Ansprüche 1 bis 9, dadurch gekennzeichnet, daß man die Ketocarbonylverbindung und den Ir-Komplex im Molverhältnis 200 bis 2000 einsetzt und die Hydrierung bei einem Druck von 0,1 bis 20 MPa, vorzugsweise 0,5 bis 12 MPa, und einer Temperatur von 10 bis 100 °C durchführt.

11. Verfahren nach einem oder mehreren der Ansprüche 1 bis 10, dadurch gekennzeichnet, daß man die Hydrierung in Gegenwart eines Lösungsmittelgemisches aus einem aromatischen Kohlenwasserstoff und einem verzweigten oder unverzweigten niederen Alkohol, vorzugsweise einem Gemisch aus Toluol oder Xylol und einem einwertigen $C_3$- bis $C_5$-Alkohol, insbesondere tert.-Butanol, durchführt.

## Claims

1. Method for the asymmetric hydrogenation of α-ketocarbonyl compounds from the group of the α-ketocarboxylic acids, α-ketocarboxylic esters, α-ketocarboxylic acid amides and particularly α-ketolactones to the corresponding optically active α-hydroxycarbonyl compounds in the presence of chiral platinum metal diphosphane complexes as catalysts, characterised in that chiral iridium complexes are used which contain an optically active 1,2-diphosphane ligand of the general formula Ia, Ib or Ic

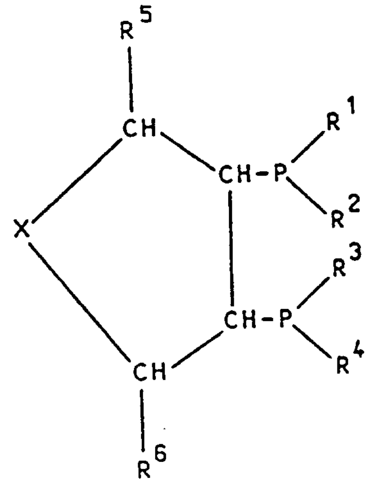

(Ia),

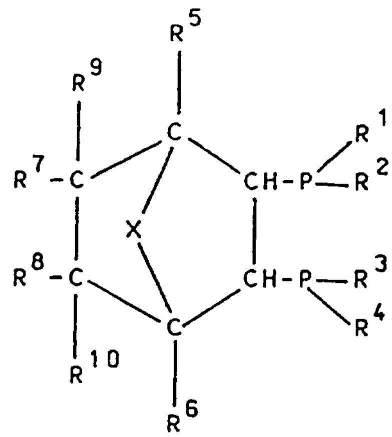

(Ib),

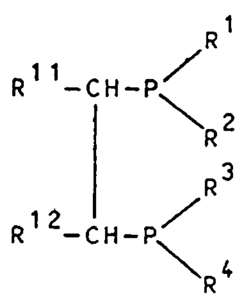

(Ic)

wherein

$R^1$, $R^2$, $R^3$ and $R^4$ are identical or different and signify $C_1$- to $C_8$-alkyl, which can be linear or branched, cyclohexylmethyl, $C_5$- to $C_7$-cycloalkyl, which can possess one or two $C_1$- to $C_4$-alkyl substituents, benzyl or phenyl, wherein the aromatic ring can contain $C_1$ to $C_4$-alkyl-, $C_1$- to $C_4$-alkoxy-, di-($C_1$- to $C_4$-) alkylamino- or $C_1$- to $C_4$-alkyloxycarbonyl substituents or $R^1$, $R^2$ or/and $R^3$, $R^4$ represent an orthobiphenylene group,

X represents oxygen, the groups $NR^{13}$, $CR^{14}R^{15}$, $-CR^{14} = CR^{15}-$ or $-CHR^{14} - CHR^{15}-$, wherein $R^{13}$ signifies hydrogen, $C_1$- to $C_6$-alkyl, $C_5$- to $C_7$-cycloalkyl, benzyl, or an acyl group from the series -CO alkyl, - CO aryl, COO alkyl, -COO aryl, $-SO_2$ aryl, -P(O) $aryl_2$, wherein the alkyl groups contain 1 to 4 C atoms, and aryl represents phenyl or naphthyl, and can contain one or two $C_1$- to $C_4$-alkyl-, $C_1$- to $C_4$-

alkoxy-, di-($C_1$- to $C_4$) alkylamino-, $C_1$- to $C_4$-alkoxycarbonyl substituents, and $R^{14}$ and $R^{15}$ are identical or different and signify hydrogen, $C_1$- to $C_4$-alkyl or phenyl,
$R^5$ and $R^6$ are identical or different and signify hydrogen, $C_1$ to $C_4$-alkyl or phenyl,
$R^7$ and $R^8$ are identical or different and signify hydrogen, phenyl, $C_1$- to $C_4$-alkoxycarbonyl or $C_1$- to $C_4$-alkyl,
$R^9$ and $R^{10}$ together represent a double bond or signify hydrogen,
$R^{11}$ and $R^{12}$ are identical or different and signify $C_1$- to $C_6$-alkyl, benzyl or phenyl, one of the radicals $R^{11}$ and $R^{12}$ can also be hydrogen or, on condition that at least one phosphorus atom or one of the radicals $R^1$ to $R^4$ is chiral, both $R^{11}$ and $R^{12}$ represent hydrogen, and the hydrogenation is conducted in the presence of a cocatalyst from the group of cyclic dicarboxylic acid imides of the general formula II

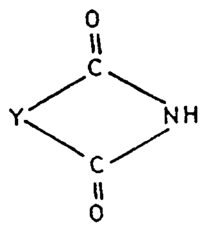 (II),

wherein Y signifies an ethylene group or tri- or tetramethylene group, which can possess a phenyl radical or one or two $C_1$- to $C_8$-alkyl substituents, as well as a vinylene group or ortho-arylene group, which can contain one or two substituents from the group ($C_1$-$C_4$)-alkyl, ($C_1$-$C_4$)-alkoxy, ($C_1$-$C_4$)-alkoxycarbonyl, di- ($C_1$-$C_4$)-alkylamino.

**2.** The method according to claim 1,
characterised in that
iridium complexes of the general formula IIIa or IIIb

$[Ir(en)_2\ (1,2\text{-}DP)]^+\ A^-$ (IIIa)

$[Ir(Z)\ (en)_2\ (1,2\ DP)]$ (IIIb)

are used as catalysts, wherein 1,2-DP signifies an optically active 1,2-diphosphane ligand of the general formula Ia, Ib or Ic, $(en)_2$ signifies two molecules of a monoolefin or one molecule of a diolefin, $A^-$ signifies a non-coordinating anion, particularly $BF_4^-$, $PF_6^-$, $ClO_4^-$ and Z signifies a coordinating anion, particularly chloride or carboxylate, which can also be present in the form of an ion exchanger.

**3.** The method according to claim 1 or 2,
characterised in that
1,2-diphosphane ligands of the general formula Ia, Ib or Ic are used, wherein $R^1 = R^3$ and $R^2 = R^4$ and preferably $R^1 = R^3 = R^2 = R^4$ and phenyl radicals are particularly preferred.

**4.** The method according to one or more of the claims 1 to 3,
characterised in that
1,2-diphosphane ligands of the general formula Ia are used, wherein $R^5$ and $R^6$ signify hydrogen and X signifies the group $NR^{13}$, wherein $R^{13}$ has the meaning given above and $R^1 = R^3$ and $R^2 = R^4$ or preferably $R^1$, $R^2$ $R^3$ and $R^4$ are identical.

**5.** The method according to one or more of the claims 1 to 3,
characterised in that
1,2-diphosphane ligands of the general formula Ib are used, wherein $R^5$, $R^6$, $R^7$ and $R^8$ signify hydrogen and X signifies a methylene group and $R^1 = R^3$ and $R^2 = R^4$ or preferably $R^1$, $R^2$ $R^3$ and $R^4$ are identical.

**6.** The method according to one or more of the claims 1 to 3,
characterised in that
1,2-diphosphane ligands of the general formula Ic are used, wherein $R^{11}$ signifies hydrogen or a methyl

group and $R^{12}$ signifies a $C_1$- to $C_4$-alkyl group, which can be linear or branched.

**7.** The method according to one or more of the claims 1 to 6,
characterised in that
succinimide, in a quantity of from 1 to 50 moles, preferably from 2 to 25 moles, per mole of iridium complex, is used as cocatalyst.

**8.** The method according to one or more of the claims 1 to 7,
characterised in that
ketopantolactone is hydrogenated to (R)-(-)-pantolactone.

**9.** The method according to one or more of the claims 1 to 8,
characterised in that
firstly the active catalyst of the general formula IIIb is prepared in situ from an Ir complex of the formula $[Ir\ (en)_2\ (Z)]_2$, wherein $(en)_2$ and Z have the meanings given above, by adding the optically active 1,2-diphosphane of the formula Ia, Ib or Ic in at least one solvent used for the hydrogenation and the ketocarbonyl compound is hydrogenated in the presence of the catalyst solution thus prepared.

**10.** The method according to one or more of the claims 1 to 9,
characterised in that
the ketocarbonyl compound and the Ir complex are used in the molar ratio of 200 to 2000 and the hydrogenation is conducted at a pressure of from 0.1 to 20 MPa, preferably from 0.5 to 12 MPa, and at a temperature of from 10 to 100 °C.

**11.** The method according to one or more of the claims 1 to 10,
characterised in that
the hydrogenation is conducted in the presence of a mixture of solvents comprising an aromatic hydrocarbon and a branched or unbranched lower alcohol, preferably a mixture of toluene or xylene and a monovalent $C_3$- to $C_5$-alcohol, in particular tert-butanol.

## Revendications

**1.** Procédé d'hydrogénation asymétrique de composés α-cétocarbonyles du groupe des acides α-cétocarboxyliques, des esters d'acides α-cétocarboxyliques, des amides d'acides α-cétocarboxyliques et notamment de l'a-cétolactone en les composés correspondants α-hydroxycarbonyle optiquement actifs en présence de complexes diphosphane chiraux de métaux du groupe du platine comme catalyseur, caractérisé en ce qu'on utilise des complexes d'iridium chiraux, qui contiennent un ligand 1,2-diphosphane optiquement actif de formule générale Ia, Ib ou Ic.

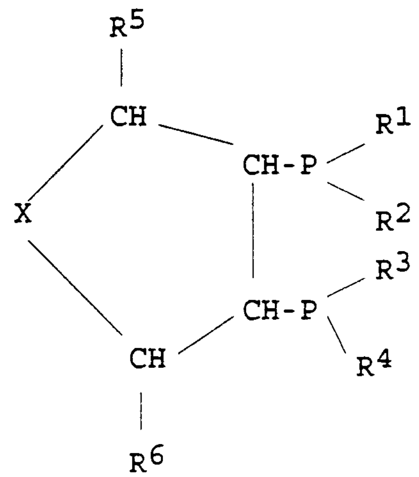

(Ia)

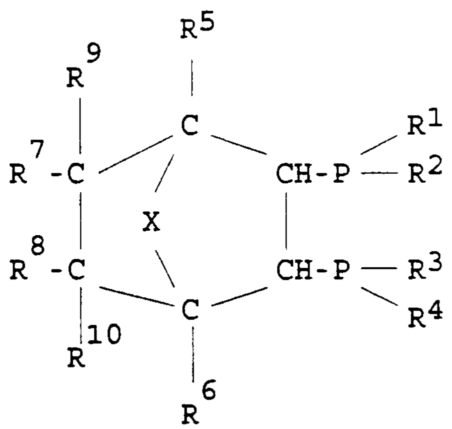

(Ib)

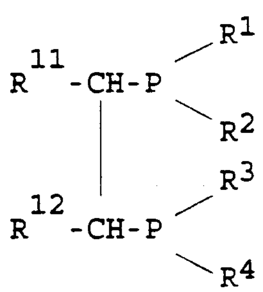

(Ic)

dans laquelle,

$R^1$, $R^2$, $R^3$ et $R^4$ sont identiques ou différents et représentent un alkyle $C_1$ à $C_8$, qui peut être linéaire ou ramifié, le cyclohexylméthyle, un cycloalkyle $C_5$ à $C_7$ qui peut présenter un ou deux substituants alkyles $C_1$ à $C_4$ , un benzyle ou phényle, le cycle aromatique peut comprendre des substituants alkyle $C_1$ à $C_4$, alcoxy $C_1$ à $C_4$, di(alkyl $C_1$ à $C_4$) amino ou (alkyl $C_1$ à $C_4$)oxycarbonyle ou $R^1$ $R^2$ et/ou $R^3$ $R^4$ représentent un groupe ortho-biphénylène,

X représente l'oxygène, le groupe $NR^{13}$, $CR^{14}$ $R^{15}$, - $CR^{14}=CR^{15}$ ou $-CHR^{14}-CHR^{15}$, dans lesquels $R^{13}$ est un hydrogène, un alkyle $C_1$ à $C_6$, un cycloalkyle $C_5$ à $C_7$, un benzyle ou un groupe acyle de la série -CO alkyle, -CO aryle, -COO alkyle, -COO aryle, $-SO_2$ aryle, $-P(O)aryle_2$, dans lesquels le groupe alkyle contient 1 à 4 atomes de C et aryle représente le naphtyle ou le phényle et peut comprendre un ou deux substituants alkyle $C_1$ à $C_4$, alcoxy $C_1$ à $C_4$, di-(alkyl $C_1$ à $C_4$)amino, (alcoxy $C_1$ à $C_4$)-carbonyle et $R^{14}$ et $R^{15}$ sont identiques ou différents et représentent l'hydrogène, un alkyle $C_1$ à $C_4$ ou un phényle,

$R^5$ et $R^6$ sont identiques ou différents et représentent un hydrogène, un alkyle $C_1$ à $C_4$ ou un phényle,

$R^7$ et $R^8$ sont identiques ou différents et représentent un hydrogène, un phényle, un (alcoxy $C_1$ à $C_4$)-carbonyle ou un alkyle $C_1$ à $C_4$.
$R^9$ et $R^{10}$ représentent en commun une double liaison ou représentent de l'hydrogène,
$R^{11}$ et $R^{12}$ sont identiques ou différents et représentent un alkyle $C_1$ à $C_6$, un benzyle ou un phényle, l'un des restes $R^{11}$ et $R^{12}$ peut être aussi de l'hydrogène ou, à la condition, qu'au moins un atome de phosphore ou l'un des restes $R^1$ à $R^4$ est chiral, $R^{11}$ et $R^{12}$ représente l'hydrogène, et on réalise l'hydrogénation en présence d'un co-catalyseur du groupe des imides cycliques d'acides dicarboxyliques de formule générale II.

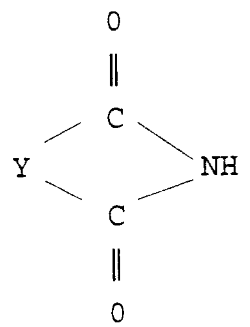

(II)

dans laquelle Y est un groupe éthylène ou tri-ou tétraméthylène, qui peuvent présenter un reste phényle ou un ou deux substituants alkyle $C_1$ à $C_8$, ainsi qu'un groupe vinylène ou ortho-arylène, qui peut présenter un ou deux substituants du groupe des alkyle($C_1$-$C_4$), alcoxy ($C_1$-$C_4$), (alcoxy $C_1$-$C_4$)-carbonyle, di-(alkyl $C_1$-$C_4$)-amino.

**2.** Procédé selon la revendication 1, caractérisé en ce qu'on utilise comme catalyseur des complexes d'iridium de formule générale IIIa ou III b

$[Ir(en)_2(1,2\text{-}DP)]^+A^-$ (IIIa)

$[Ir(Z)(en)_2(1,2\ DP)]$ (IIIb)

dans lesquelles 1,2-DP représente un ligand optiquement actif de 1,2-diphosphane de formule générale Ia, Ib ou Ic, $(en)_2$ deux molécules d'une mono-oléfine ou une molécule d'une dioléfine, $A^-$ un anion non coordinant notamment $BF_4^-$, $PF_6^-$, $ClO_4^-$ et Z un anion coordinant, notamment du chlorure ou un carboxylate, qui peut se présenter aussi sous forme d'un échangeur d'ions.

**3.** Procédé selon la revendication 1 ou 2, caractérisé en ce qu'on utilise un ligand 1,2-diphosphane de formule générale Ia, Ib ou Ic dans lesquels $R^1 = R^3$ et $R^2 = R^4$ et de préférence $R^1 = R^2 = R^3 = R^4$ et ce sont de préférence des restes phényles

**4.** Procédé selon l'une ou plusieurs des revendications 1 à 3, caractérisé en ce qu'on utilise des ligands de 1,2-diphosphane de formule générale Ia, dans laquelle $R^5$ et $R^6$ représentent l'hydrogène et X le groupe $NR^{13}$, $R^{13}$ ayant la signification déjà donnée et $R^1 = R^3$ et $R^2 = R^4$ ou de préférence $R^1$, $R^2$, $R^3$ et $R^4$ sont identiques.

**5.** Procédé selon l'une ou plusieurs des revendications 1 à 3, caractérisé en ce qu'on utilise des ligands 1,2-diphosphane de formule générale Ib dans lesquels $R^5$, $R^6$, $R^7$ et $R^8$ représentent un hydrogène et X un groupe méthylène et $R^1 = R^3$ et $R^2 = R^4$ ou de préférence $R^1$, $R^2$, $R^3$ et $R^4$ sont identiques.

**6.** Procédé selon l'une ou plusieurs des revendications 1 à 3, caractérisé en ce qu'on utilise des ligands 1,2-diphosphane de formule générale Ic, dans laquelle $R^{11}$ représente un hydrogène ou un groupe méthyle et $R^{12}$ un groupe alkyle $C_1$ à $C_4$, qui peut être linéaire ou ramifié.

**7.** Procédé selon l'une ou plusieurs des revendications 1 à 4, caractérisé en ce qu'on utilise comme cocatalyseur du succinimide en une quantité de 1 à 50 moles, de préférence 2 à 25 moles par mole de complexe d'iridium.

8. Procédé selon l'une ou plusieurs des revendications 1 à 7, caractérisé en ce qu'on hydrogène la cétopantolactone en (R)-(-)- pantolactone.

9. Procédé selon l'une ou plusieurs des revendication 1 à 8, caractérisé en ce qu'on prépare d'abord in situ le catalyseur efficace de formule générale IIIb à partir d'un complexe Ir de formule $[Ir(en)_2(Z)]_2$ dans lequel $(en)_2$ et Z ont la signification indiquée, par addition du 1,2-diphosphane actif de formule Ia, Ib ou Ic dans au moins un solvant utilisable pour l'hydrogénation et on hydrogène le composé cétocarbonyle en présence de la solution de catalyseur ainsi obtenue.

10. Procédé selon l'une ou plusieurs des revendication 1 à 9, caractérisé en ce qu'on utilise le composé cétocarbonyle et le complexe Ir en proportion molaire de 200 à 2000 et on réalise l'hydrogénation sous une pression de 0,1 à 20 MPa, de préférence 0,5 à 12 MPa et à une température de 10 à 100 ° C.

11. Procédé selon l'une ou plusieurs des revendication 1 à 10, caractérisé en ce qu'on réalise l'hydrogénation en présence d'un mélange de solvant d'un hydrocarbure aromatique et d'un alcool inférieur ramifié ou non, de préférence un mélange de toluène ou de xylène et d'un monoalcool $C_3$ à $C_5$, notamment le tert. butanol.